# EUROPÄISCHE PATENTANMELDUNG

(11) **EP 4 325 224 A1**
(43) Veröffentlichungstag der Anmeldung: **21.02.2024**
(21) Anmeldenummer: 22190892.4
(22) Anmeldetag: 18.08.2022
(51) Int. Cl.: G01N 33/86

(54) **THROMBOZYTENAKTIVIERUNGSTEST ZUR DIAGNOSE EINER HEPARIN-INDUZIERTEN THROMBOZYTOPENIE**

(71) Anmelder: Siemens Healthcare Diagnostics Products GmbH, 35041 Marburg (DE)
(72) Erfinder: Schwarz, Herbert, 35102 Lohra (DE); Christ, Gerlinde, 35043 Marburg (DE); Ehm, Matthias, 35094 Lahntal/ Sterzhausen (DE)
(74) Vertreter: Siemens Healthineers Patent Attorneys

(57) **Zusammenfassung**

Die vorliegende Erfindung betrifft einen funktionellen, leicht automatisierbaren Test zur Feststellung einer Heparininduzierten Thrombozytopenie (HIT). Es wird die Entstehung von Prothrombinfragment F1+2 im Reaktionsansatz gemessen.

## Beschreibung

Die vorliegende Erfindung liegt auf dem Gebiet der in vitro-Diagnostik und betrifft einen funktionellen, leicht automatisierbaren Test zur Feststellung einer Heparin-induzierten Thrombozytopenie.

Die Heparin-induzierte Thrombozytopenie (HIT) ist eine thrombotische Erkrankung, die im Rahmen einer Heparintherapie entstehen kann und lebensbedrohliche thromboembolische Komplikationen verursachen kann. Betroffene Patienten produzieren Antikörper, die einen Komplex aus Heparin und Plättchenfaktor 4 (PF4) binden, sogenannte anti-PF4/Heparin-Komplex-Antikörper. In vivo bindet der Antikörper-gebundene PF4/Heparin-Komplex an die Thrombozytenoberfläche und verursacht eine Aktivierung der Thrombozyten. Dadurch kommt es zu einer Abnahme der Thrombozytenzahl und einem erhöhten Risiko für Thromboembolien.

Zur HIT-Diagnose werden im Wesentlichen zwei Testprinzipien angewendet. Das erste Testprinzip beruht auf dem direkten Nachweis von anti-PF4/Heparin-Komplex-Antikörpern in einer Körperflüssigkeitsprobe eines Patienten. Dazu wird eine Probe mit PF4/Heparin-Komplex oder einem Komplex aus PF4 und einem anderen geeigneten Polyanion (wie z.B. Polyvinylsulfonat) in Kontakt gebracht, und die Bindung etwaiger in der Probe vorhandener anti-PF4/Heparin-Komplex-Antikörper mit herkömmlichen immunologischen Testverfahren (z.B. ELISA) nachgewiesen. Nachteilig ist jedoch, dass der Nachweis von anti-PF4/Heparin-Komplex-Antikörpern zwar sensitiv, aber nicht ausreichend spezifisch ist, d.h. der Nachweis der Antikörper ist nicht hinreichend für eine positive HIT-Diagnose, während ein negatives Ergebnis eine HIT angemessen sicher ausschließt. Daher wird die Bestätigung durch einen funktionellen Test basierend auf einem zweiten Testprinzip empfohlen.

Das zweite, funktionelle Testprinzip beruht auf dem Nachweis der Thrombozyten-aktivierenden Wirkung der anti-PF4/Heparin-Komplex-Antikörper. Bei diesem Testprinzip werden gewaschene Thrombozyten von einem oder mehreren normalen Spendern mit einer Plasma- oder Serumprobe eines Patienten und mit Heparin gemischt, und es wird die Thrombozytenaktivierung anhand bekannter Aktivierungsmarker, wie z.B. anhand der Menge von freigesetztem Serotonin (Serotoninfreisetzungstest), oder anhand der visuell erkennbaren Aggregationsreaktion der Thrombozyten (HIPA-Test) gemessen. Enthält eine Patientenprobe anti-PF4/Heparin-Komplex-Antikörper ist eine gegenüber einer normalen Probe (ohne derartige Antikörper) erhöhte Thrombozytenaktivierung feststellbar.

Die beiden genannten funktionellen Testprinzipien können bislang nicht in herkömmlichen Laboren und nicht automatisiert durchgeführt werden, weil sie eine komplexe manuelle Durchführung erfordern und daher nur von geschultem Personal ausgeführt werden können. Darüber hinaus erfordert der Serotoninfreisetzungstest die Abtrennung der Thrombozyten durch einen Zentrifugationsschritt und die Verwendung von radioaktivem Material.

In EP 3413051 A1 ist ein anderes Verfahren zur Detektion von anti-PF4/Heparin-Komplex-Antikörpern in einer Körperflüssigkeitsprobe beschrieben, welches folgende Schritte umfasst:
i. Bereitstellen eines Reaktionsgemisches durch Vermischen der Probe mit einem Thrombozyten-haltigen Reagenz und mit einem PF4-bindenden, unverzweigten Polysaccharid oder mit einem PF4-bindenden Polyanion;
ii. Inkubation des Reaktionsgemisches; und dann
iii. Bestimmen der Menge von PF4 in dem Reaktionsgemisch;
iv. Vergleichen der so bestimmten Menge von PF4 in dem Reaktionsgemisch mit einem vorbestimmten Referenzwert für die Menge von PF4 in Reaktionsgemischen enthaltend Körperflüssigkeitsproben von Spendern, die bekanntermaßen keine anti-PF4/Heparin-Komplex-Antikörper enthalten; und
v. Feststellen des Vorhandenseins von anti-PF4/Heparin-Komplex-Antikörpern in der Probe, wenn die in dem Reaktionsgemisch bestimmte Menge von PF4 den Referenzwert überschreitet.

Durch die quantitative Bestimmung von freigesetztem PF4 in einem Reaktionsgemisch, welches die zu untersuchende Probe, Thrombozyten und Heparin (oder ein funktionell gleichwertiges, PF4-bindendes Polysaccharid oder Polyanion) enthält, kann festgestellt werden, ob die Probe anti-PF4/Heparin-Komplex-Antikörper enthält und der Patient damit an einer Heparin-induzierte Thrombozytopenie leidet.

Ein Nachteil der vorgenannten Verfahren ist, dass mit der Detektion des Vorhandenseins von anti-PF4/Heparin-Komplex-Antikörpern, die eine Thrombozytenaktivierung verursachen, zwar das Vorliegen einer Heparin-induzierte Thrombozytopenie bestimmt werden kann, dass aber ein prokoagulatorischer Status des Patienten, also eine bereits tatsächlich erhöhte Gerinnungsaktivität, die einen Patienten auszeichnet, der ein besonders hohes, akutes Risiko an einem thrombotischem Ereignis zu erkranken aufweist, nicht immer erkannt wird.

Der vorliegenden Erfindung liegt daher die Aufgabe zugrunde ein funktionelles Verfahren zur Detektion von anti-PF4/Heparin-Komplex-Antikörpern in einer Körperflüssigkeitsprobe bereit zu stellen, das neben der Detektion des bloßen Vorhandenseins von anti-PF4/Heparin-Komplex-Antikörpern und der damit verbundenen Bestimmung einer Heparin-induzierten Thrombozytopenie, zusätzlich Hinweise auf einen -höchstwahrscheinlich durch die von den anti-PF4/Heparin-Komplex-Antikörpern induzierte Thrombozytenaktivierung verursachten- prokoagulatorischen Status des Patienten liefert.

Es wurde gefunden, dass durch die quantitative Bestimmung von Prothrombinfragment F1+2 (F1+2) in einem Reaktionsgemisch, welches die zu untersuchende Probe, Thrombozyten und Heparin (oder ein funktionell gleichwertiges, PF4-bindendes Polysaccharid oder Polyanion) enthält, einerseits festgestellt werden kann, ob die Probe anti-PF4/Heparin-Komplex-Antikörper enthält und damit eine Heparin-induzierten Thrombozytopenie vorliegt. Proben, die anti-PF4/Heparin-Komplex-Antikörper enthalten, verursachen in dem genannten Reaktionsgemisch offenbar die Entstehung von Thrombin, so dass eine erhöhte Menge des bei der Entstehung von Thrombin entstehenden Prothrombinfragments F1+2 in dem Reaktionsgemisch nicht nur das Vorhandensein von anti-PF4/Heparin-Komplex-Antikörpern anzeigt und damit das Vorliegen einer Heparin-induzierten Thrombozytopenie, sondern zusätzlich eine Bewertung des prokoagulatorischen Status des Patienten erlaubt. Je größer die Menge des im Reaktionsansatz generierten Prothrombinfragments F1+2, desto größer ist die prokoagulatorische Aktivität und damit das akute Risiko des Patienten an einem thrombotischem Ereignis zu erkranken. Dies ermöglicht die Identifikation besonders gefährdeter Patienten.

Gegenstand der vorliegenden Erfindung ist also ein Verfahren zur Detektion von anti-PF4/Heparin-Komplex-Antikörpern in einer Körperflüssigkeitsprobe. Das Verfahren umfasst die Schritte:
i. Bereitstellen eines Reaktionsgemisches durch Vermischen der Probe mit einem Thrombozyten-haltigen Reagenz und mit einem PF4-bindenden, unverzweigten Polysaccharid oder mit einem PF4-bindenden Polyanion oder mit einem Komplex bestehend aus PF4-Protein gebunden an ein unverzweigtes Polysaccharid oder an ein Polyanion;
ii. Inkubation des Reaktionsgemisches; und dann
iii. Bestimmen der Menge von Prothrombinfragment F1+2 (F1+2) in dem Reaktionsgemisch;
iv. Vergleichen der so bestimmten Menge von F1+2 in dem Reaktionsgemisch mit einem vorbestimmten Referenzwert für die Menge von F1+2 in Reaktionsgemischen enthaltend Körperflüssigkeitsproben von Spendern, die bekanntermaßen keine anti-PF4/Heparin-Komplex-Antikörper enthalten; und
v. Feststellen des Vorhandenseins von anti-PF4/Heparin-Komplex-Antikörpern in der Probe, wenn die in dem Reaktionsgemisch bestimmte Menge von F1+2 den Referenzwert überschreitet.

Die Körperflüssigkeitsprobe stammt bevorzugterweise von einem Menschen. Bevorzugterweise handelt es sich um eine im Wesentlichen Thrombozyten-freie Körperflüssigkeitsprobe, insbesondere um Plasma.

Das für die Bereitstellung des Reaktionsgemisches benötigte Thrombozyten-haltige Reagenz ist vorzugsweise eine Suspension gewaschener und resuspendierter humaner Thrombozyten. Die Thrombozyten stammen vorzugsweise von einem oder mehreren gesunden Spendern, die bekanntermaßen keine anti-PF4/Heparin-Komplex-Antikörper enthalten. Zur Herstellung eines geeigneten Thrombozyten-haltigen Reagenzes werden beispielsweise Citrat-Vollblutproben, denen vorzugsweise auch Hirudin zugegeben wurde, zentrifugiert, um plättchenreiches Plasma zu gewinnen. Dem plättchenreichen Plasma wird nochmals Citratlösung und Apyrase zugesetzt, es wird nochmals zentrifugiert, und anschließend wird der zellfreie Überstand verworfen. Die pelletierten Thrombozyten werden schließlich in einer gepufferten Suspensionslösung aufgenommen.

Die Körperflüssigkeitsprobe wird ferner mit einem PF4-bindenden, unverzweigten Polysaccharid oder mit einem PF4-bindenden Polyanion vermischt. Es sind eine Vielzahl PF4-bindender Substanzen bekannt, die mit PF4 einen Komplex ausbilden, der von den zu detektierenden anti-PF4/Heparin-Komplex-Antikörpern gebunden wird. Geeignete unverzweigte Polysaccharide sind beispielsweise Heparin, unfraktioniertes Heparin (UFH), fraktioniertes Heparin (LMWH), Dextransulfat und Fucoidan. Geeignete Polyanionen sind beispielsweise Polyvinylsulfat, Polyvinylsulfonat, Polyvinylphosphat, Polyvinylphosphonat, Polystyrensulfat und Polystyrensulfonat.

Besonders bevorzugt ist die Verwendung von niedermolekularen Dextransulfaten, vorzugsweise von Dextransulfaten mit einem Molekulargewicht von 6 bis 10 Kilodalton (kDa), da sie ein Minimum unerwünschter Nebeneffekte, wie z.B. Aktivierung des intrinsischen Gerinnungssystems oder Abschwächung der Thrombozytenadhäsion, garantieren.

In einer anderen Ausführungsform des erfindungsgemäßen Verfahrens wird die Körperflüssigkeitsprobe alternativ mit einem Komplex bestehend aus PF4-Protein gebunden an ein unverzweigtes Polysaccharid (vorzugsweise an eines der obengenannten) oder an ein Polyanion (vorzugsweise an eines der obengenannten) vermischt.

Vor der Bestimmung der F1+2-Menge in dem Reaktionsgemisch wird das Reaktionsgemisch für einen Zeitraum inkubiert, um die Komplexbildung zwischen dem PF4-bindenden, unverzweigten Polysaccharid oder Polyanion und dem in der Probe vorhandenen PF4-Protein, die Bindung der vermutlich in der Probe enthaltenen anti-PF4/Heparin-Komplex-Antikörper an den entstandenen (oder alternativ an den zugegebenen) Komplex und schließlich die Aktivierung der Thrombozyten durch die Bindung des Antikörper-gebundenen Komplexes an der Thrombozytenoberfläche zu ermöglichen. Eine typische Inkubationsdauer beträgt zwischen 5 und 15 Minuten, vorzugsweise bei +37 °C.

Nach ausreichender Inkubation werden Schritte durchgeführt, um die Menge von F1+2 in dem Reaktionsgemisch zu bestimmen. Die Bestimmung der Menge von F1+2 in dem Reaktionsgemisch kann auf unterschiedliche Art und Weise erfolgen. Bevorzugt sind Bindungsteste, bei denen zum Bestimmen der Menge von F1+2 in dem Reaktionsgemisch mindestens ein F1+2-spezifischer Bindungspartner, wie z.B. ein anti-F1+2-spezifischer Antikörper, mit dem Reaktionsgemisch in Kontakt gebracht wird. Dies kann beispielsweise dadurch erfolgen, dass das Reaktionsgemisch (ganz oder teilweise) mit einem an eine Festphase assoziierten F1+2-Bindungspartner in Kontakt gebracht wird. Ungebundene Bestandteile werden durch Waschen entfernt, und mit Hilfe eines zweiten, markierten F1+2-Bindungspartners wird die Menge des an die Festphase gebundenen F1+2-Peptids bestimmt (ELISA-Technik). Besonders geeignete anti-F1+2-Antikörper sind monoklonale Fl+2-spezifische Antikörper, die nicht an ungespaltenes Prothrombin binden. Die Herstellung und Verwendung solcher spezifischer F1+2-Antikörper, die nicht an Prothrombin binden, ist z.B. in EP 303983 A2 (Pelzer et al.), in US 5,830,681 (Hursting et al.) oder in US 2003/0219845 A1 (Ruiz et al.) beschrieben. Für die Spezifität der anti-F1+2-Antikörper ist es wichtig, dass diese an ein Epitop binden, welches zumindest die vier carboxyterminalen Aminosäuren des F1+2-Fragments (Ile-Glu-Gly-Arg-OH) enthält. Da in der Regel zur Bestimmung der F1+2-Konzentration Sandwich-Immunoassays eingesetzt werden, werden zwei anti-F1+2-Antikörper benötigt. In EP 1541676-A1 (Teigelkamp et al.) sind Antikörper beschrieben, die an ein Epitop auf der N-terminalen Hälfte des F1+2-Fragments und damit auch an intaktes Prothrombin binden, sich jedoch zur Verwendung als Sekundärantikörper in Kombination mit den F1+2-Neoepitop-spezifischen Primärantikörpern in einem Sandwich-Immunoassay besonders eignen.

Besonders bevorzugt sind homogene Immunteste, mit denen auf die Entfernung ungebundener Bestandteile und damit auf Waschschritte verzichtet werden kann. Dazu wird das Reaktionsgemisch (ganz oder teilweise) mit einem ersten und einem zweiten anti-F1+2-Antikörper und mit einer ersten und einer zweiten Komponente eines signalbildenden Systems vermischt. Die Komponenten des signalbildenden Systems wirken derart zusammen, dass ein detektierbares Signal entsteht, wenn die erste und die zweite Komponente des signalbildenden Systems in räumliche Nähe zueinander gebracht werden. Dabei ist der erste anti-F1+2-Antikörper mit der ersten Komponente des signalbildenden Systems assoziiert oder wird während der Inkubation des Reaktionsgemisches damit assoziiert, und der zweite anti-F1+2-Antikörper ist mit der zweiten Komponente des signalbildenden Systems assoziiert oder wird während der Inkubation des Reaktionsgemisches damit assoziiert.

In einer anderen Ausführungsform des erfindungsgemäßen Verfahrens umfasst der F1+2-spezifische Immunoassay die Verwendung eines ersten Antikörpers mit Spezifität für F1+2 und die Verwendung eines zweiten Antikörpers mit Spezifität für den Immunkomplex bestehend aus F1+2 und dem ersten Antikörper.

Ein derartiger F1+2-spezifischer Immunoassay ist in
EP 2168985-A1 (Althaus et al.) beschrieben. Dabei wird ein Antikörper verwendet, der spezifisch an einen Immunkomplex bindet, welcher Prothrombin-Fragment F1+2, an welches ein Antikörper oder ein Antikörperfragment mit Spezifität für das carboxyterminale Neoepitop des Prothrombin-Fragmentes F1+2 gebunden ist, enthält und wobei der Antikörper aber nicht an das Prothrombin-Fragment F1+2 oder F2 alleine und nicht an den Antikörper oder das Antikörperfragment mit Spezifität für das carboxyterminale Neoepitop des Prothrombin-Fragmentes F2/F1+2 alleine bindet. Dieses Assayformat gewährleistet eine so hohe Spezifität, dass es die Durchführung eines direkten, homogenen Sandwich-Immunoassays ohne Wasch- oder Trennschritte ermöglicht.

Bei einer Ausführungsform eines homogenen Fl+2-Immuntestes handelt es sich bei den Komponenten des signalbildenden Systems um partikuläre Festphasen, beispielsweise Latexpartikel, deren Agglutination turbidimetrisch oder nephelometrisch bestimmt wird. Dazu besteht die erste Komponente des signalbildenden Systems aus einer ersten partikulären Festphase, die so beschaffen ist, dass sie mit dem ersten anti-F1+2-Antikörper assoziiert ist oder assoziierbar ist. Die erste partikuläre Festphase kann mit dem ersten anti-F1+2-Antikörper über eine kovalente Bindung oder über ein Bindungspaar X/Y verbunden sein oder über ein Bindungspaar X/Y im Reaktionsansatz verbunden werden. Weiterhin besteht die zweite Komponente des signalbildenden Systems aus einer zweiten partikulären Festphase, die so beschaffen ist, dass sie mit dem zweiten anti-F1+2-Antikörper (oder mit dem Antikörper mit Spezifität für den Immunkomplex bestehend aus F1+2 und dem ersten Antikörper) assoziiert ist oder assoziierbar ist. Die zweite partikuläre Festphase kann mit dem zweiten anti-F1+2-Antikörper (oder mit dem Immunkomplex-spezifischen Antikörper) über eine kovalente Bindung oder über ein Bindungspaar A/B verbunden sein oder über ein Bindungspaar A/B im Reaktionsansatz verbunden werden. Immunoassays beruhend auf dem Prinzip der partikelverstärkten Lichtstreuung sind seit etwa 1920 bekannt (zur Übersicht siehe Newman, D.J. et al., Particle enhanced light scattering immunoassay. Ann Clin Biochem 1992; 29: 22-42). Bevorzugterweise werden Polystyrolpartikel mit einem Durchmesser von 0,1 bis 0,5 µm, besonders bevorzugt mit einem Durchmesser von 0,15 bis 0,35 µm verwendet. Bevorzugt werden Polystyrolpartikel mit Amin-, Carboxyl- oder Aldehydfunktionen verwendet. Weiterhin bevorzugt werden Schale/Kern-Partikel verwendet. Die Synthese der Partikel und die kovalente Kopplung von Liganden ist z.B. in Peula, J.M. et al., Covalent coupling of antibodies to aldehyde groups on polymer carriers. Journal of Materials Science: Materials in Medicine 1995; 6: 779-785 beschrieben.

In einer anderen Ausführungsform eines homogenen PF4-Immuntestes umfasst das signalbildende System mindestens eine erste und eine zweite Komponente, welche so zusammenwirken, dass ein detektierbares Signal entsteht, wenn sie in räumliche Nähe zueinander gebracht werden und dadurch miteinander in Wechselwirkung treten können. Unter einer Wechselwirkung zwischen den Komponenten ist insbesondere ein Energietransfer - also die direkte Übertragung von Energie zwischen den Komponenten, z.B. durch Licht- oder Elektronenstrahlung sowie über reaktive chemische Moleküle, wie z. B. kurzlebigen Singulett-Sauerstoff - zu verstehen. Der Energietransfer kann von einer auf eine andere Komponente erfolgen, möglich ist aber auch eine Kaskade verschiedener Substanzen über die der Energietransfer läuft. Zum Beispiel kann es sich bei den Komponenten um ein Paar aus einem Energiespender und einem Energieempfänger handeln, wie beispielsweise Photosensitizer und chemilumineszierendes Agens (EP-A2-0515194, LOCI^{®} Technologie) oder Photosensitizer und Fluorophor (WO 95/06877) oder radioaktives Iod<125> und Fluorophor (Udenfriend et al. (1985) Proc. Natl. Acad. Sci. 82: 8672 - 8676) oder Fluorophor und Fluoreszenz-Quencher (US 3,996,345). Besonders bevorzugt ist die erste Komponente des signalbildenden Systems ein chemilumineszierendes Agens und die zweite Komponente des signalbildenden Systems ein Photosensitizer oder umgekehrt, und es wird die Chemilumineszenz im Reaktionsgemisch gemessen.

Die erste Komponente und/oder die zweite Komponente des signalbildenden Systems, die miteinander in Wechselwirkung treten können, können mit einer partikulären Festphase kovalent oder über spezifische Wechselwirkung assoziiert oder in diese eingelagert sein. Unter dem Begriff partikuläre Festphase sind nicht-zelluläre, suspendierbare Teilchen zu verstehen, wie z.B. Metallsolen, Silica-Partikel, Magnetpartikel oder besonders bevorzugt Latexpartikel. Bevorzugt werden Partikel mit einem Durchmesser von 0,01 - 10 µm, insbesondere bevorzugt werden Partikel mit einem Durchmesser von 0,1 - 1 µm.

Die erste Komponente des signalbildenden Systems, dessen Komponenten so zusammenwirken, dass ein detektierbares Signal entsteht, wenn sie in räumliche Nähe zueinander gebracht werden und dadurch miteinander in Wechselwirkung treten können, ist so beschaffen, dass sie mit dem ersten anti-F1+2-Antikörper assoziiert ist oder assoziierbar ist. Die erste Komponente des signalbildenden Systems kann direkt mit dem ersten anti-F1+2-Antikörper assoziiert sein oder assoziierbar sein. Bevorzugterweise ist die erste Komponente des signalbildenden Systems indirekt mit dem ersten anti-F1+2-Antikörper assoziiert oder assoziierbar. Dazu ist die erste Komponente des signalbildenden Systems mit einer partikulären Festphase assoziiert, welche zusätzlich mit dem ersten anti-F1+2-Antikörper kovalent oder über ein Bindungspaar X/Y assoziiert ist oder über ein Bindungspaar X/Y assoziierbar ist.

Die zweite Komponente des signalbildenden Systems, dessen Komponenten so zusammenwirken, dass ein detektierbares Signal entsteht, wenn sie in räumliche Nähe zueinander gebracht werden und dadurch miteinander in Wechselwirkung treten können, ist so beschaffen, dass sie mit dem zweiten anti-PF4-Antikörper (oder dem Immunkomplex-spezifischen Antikörper) assoziiert ist oder assoziierbar ist. Die zweite Komponente des signalbildenden Systems kann direkt mit dem zweiten anti-F1+2-Antikörper (oder dem Immunkomplex-spezifischen Antikörper) assoziiert sein oder assoziierbar sein. Bevorzugterweise ist die zweite Komponente des signalbildenden Systems indirekt mit dem zweiten anti-F1+2-Antikörper (oder dem Immunkomplex-spezifischen Antikörper) assoziiert oder assoziierbar. Dazu ist die zweite Komponente des signalbildenden Systems mit einer partikulären Festphase assoziiert, welche zusätzlich mit dem Liganden kovalent oder über ein Bindungspaar A/B assoziiert ist oder über ein Bindungspaar A/B assoziierbar ist.

Die "Bindungspartner X und Y" beziehungsweise die "Bindungspartner A und B" sind jeweils zwei unterschiedliche Moleküle, die einander spezifisch erkennen und binden. Beispiele für spezifische Erkennung und Bindung sind Antikörper-Antigen-Wechselwirkungen, Polynukleotid-Wechselwirkungen etc.

Geeignete Bindungspaare X/Y beziehungsweise A/B sind vor allem Antigen/Antikörper-Kombinationen, wobei der Bindungspartner X oder A ein antigenes Epitop des anti-PF4-Antikörpers ist. Das antigene Epitop kann ein natürliches Sequenz- oder Strukturepitop des Antikörpers sein. Das antigene Epitop kann auch ein heterologes Sequenz- oder Strukturepitop eines modifizierten anti-PF4-Antikörpers sein. Beispiele für heterologe Sequenz- oder Strukturepitope sind FLAG- oder HIS- oder Fluorescein-Tags, die insbesondere zur Markierung von Peptiden oder Proteinen verwendet werden. Weitere geeignete Bindungspaare X/Y beziehungsweise A/B sind komplementäre Polynukleotide X und Y beziehungsweise A und B. Besonders bevorzugte Bindungspaare X/Y beziehungsweise A/B sind FLAG-Tag/anti-FLAG-Tag-Antikörper, HIS-Tag/anti-HIS-Tag-Antikörper Fluorescein/anti-Fluorescein-Antikörper, Biotin/Avidin und Biotin/Streptavidin.

Es wurde gefunden, dass mit den beschriebenen homogenen Immuntesten auf einen Zentrifugationsschritt zur Abtrennung der Thrombozyten in dem Reaktionsgemisch verzichtet werden kann. Dies stellt eine Vereinfachung des Verfahrensablaufs dar, wodurch eine automatische Abarbeitung des Verfahrens in gängigen Analysegeräten ermöglicht wird. In einer bevorzugten Ausführungsform des erfindungsgemäßen Verfahrens wird das bereitgestellte Reaktionsgemisch keinem Zentrifugationsschritt zur Sedimentation oder Abtrennung der Thrombozyten unterworfen.

Nachdem die F1+2-Menge in dem Reaktionsgemisch bestimmt wurde, wird die so bestimmte Menge von F1+2 mit einem vorbestimmten Referenzwert verglichen. Als Referenzwert eignet sich die Menge von F1+2, die mit demselben Verfahren in Reaktionsgemischen bestimmt wird (oder vorab bestimmt wurde), die Körperflüssigkeitsproben von Spendern enthalten, die bekanntermaßen keine anti-PF4/Heparin-Komplex-Antikörper enthalten. Üblicherweise wird zur Bestimmung eines Referenzwertes in einer Vielzahl von Proben von gesunden Spendern, die bekanntermaßen keine anti-PF4/Heparin-Komplex-Antikörper oder andere Blutgerinnungsstörungen aufweisen, die F1+2-Menge bestimmt und dann mit der F1+2-Menge einer Vielzahl von Proben von an HIT erkrankten Spendern, die anti-PF4/Heparin-Komplex-Antikörper aufweisen, verglichen. Ein Referenzwert kann beispielsweise dann ein Grenzwert sein, der die Differenzierung von Proben mit und Proben ohne anti-PF4/Heparin-Komplex-Antikörpern ermöglicht. Überschreitet die in einem Reaktionsgemisch bestimmte Menge von F1+2 den Referenzwert, erlaubt dies das Feststellen des Vorhandenseins von anti-PF4/Heparin-Komplex-Antikörpern in der Probe. Wenn die in dem Reaktionsgemisch bestimmte Menge von F1+2 den Referenzwert hingegen unterschreitet, erlaubt dies das Feststellen des Fehlens von anti-PF4/Heparin-Komplex-Antikörpern in der Probe.

Ein weiterer Gegenstand der vorliegenden Erfindung ist ein Verfahren zur Diagnose oder Prognose einer Heparin-induzierten Thrombozytopenie, wobei mit einem erfindungsgemäßen Verfahren das Vorhandensein von anti-PF4/Heparin-Komplex-Antikörpern in einer Körperflüssigkeitsprobe eines Patienten detektiert wird.

Noch ein weiterer Gegenstand der vorliegenden Erfindung ist ein Testkit zur Durchführung eines erfindungsgemäßen Verfahrens. Das Testkit enhält mindestens folgende Komponenten:
a. ein erstes Reagenz enthaltend Thrombozyten;
b. ein zweites Reagenz enthaltend ein PF4-bindendes, unverzweigtes Polysaccharid oder ein PF4-bindendes Polyanion oder einen Komplex bestehend aus PF4-Protein gebunden an ein unverzweigtes Polysaccharid oder an ein Polyanion; und
c. ein oder mehrere Reagenzien zum Nachweis von F1+2, wobei mindestens ein Reagenz einen anti-F1+2-spezifischen-Antikörper enthält.

Das Reagenz enthaltend Thrombozyten ist vorzugsweise eine Suspension gewaschener und resuspendierter humaner Thrombozyten. Die Thrombozyten stammen vorzugsweise von einem oder mehreren gesunden Spendern, die bekanntermaßen keine anti-PF4/Heparin-Komplex-Antikörper enthalten. Zur Herstellung eines geeigneten Thrombozyten-haltigen Reagenzes werden beispielsweise Citrat-Vollblutproben, denen vorzugsweise auch Hirudin zugegeben wurde, zentrifugiert, um plättchenreiches Plasma zu gewinnen. Dem plättchenreichen Plasma wird nochmals Citratlösung und Apyrase zugesetzt, es wird nochmals zentrifugiert, und anschließend wird der zellfreie Überstand verworfen. Die pelletierten Thrombozyten werden schließlich in einer gepufferten Suspensionslösung aufgenommen. Ein geeignetes Thrombozyten-haltiges Reagenz enthält mindestens 300 x 10⁹ Thrombozyten pro Liter.

Das zweite Reagenz enthält entweder
- ein PF4-bindendes, unverzweigtes Polysaccharid, bevorzugt aus der Gruppe Heparin, unfraktioniertes Heparin, fraktioniertes Heparin, Dextransulfat und Fucoidan; oder
- ein PF4-bindendes Polyanion, bevorzugt aus der Gruppe Polyvinylsulfat, Polyvinylsulfonat, Polyvinylphosphat, Polyvinylphosphonat, Polystyrensulfat und Polystyrensulfonat; oder
- einen Komplex bestehend aus PF4-Protein gebunden an eines der vorgenannten unverzweigten Polysaccharide oder bestehend aus PF4-Protein gebunden an eines der vorgenannten Polyanionen.

Das erste und das zweite Reagenz sind zur Bereitstellung des Reaktionsgemisches mit der Körperflüssigkeitsprobe vorgesehen.

Das Testkit enthält außerdem ein oder mehrere Reagenzien zum Nachweis von F1+2, wobei mindestens ein Reagenz einen anti-F1+2-spezifischen-Antikörper enthält. Bei einem Reagenz, das einen anti-F1+2-spezifischen-Antikörper enthält, kann es sich um einen Festphasen-assoziierten Antikörper handeln, beispielsweise um einen an Latexpartikel oder in der Vertiefung einer Mikrotitrationsplatte gebundenen Antikörper. Je nach Testformat enthält ein Testkit zusätzliche Komponenten zum quantitativen Nachweis von anti-F1+2-Antikörper-gebundenem F1+2, wie z.B. ein Reagenz enthaltend einen mit einem nachweisbaren Label markierten Zweitantikörper.

Ein bevorzugtes Testkit enthält ein Reagenz enthaltend einen ersten anti-F1+2-spezifischen Antikörper und ein anderes Reagenz enthaltend einen zweiten anti-F1+2-Antikörper (oder einen Antikörper mit Spezifität für den Immunkomplex bestehend aus F1+2 und dem ersten Antikörper). Der erste und/oder der zweite anti-F1+2-Antikörper können mit einer Festphase und/oder einer ersten beziehungsweise zweiten Komponente eines signalbildenden Systems, dessen Komponenten so zusammenwirken, dass ein detektierbares Signal entsteht, wenn sie in räumliche Nähe zueinander gebracht werden, assoziiert sein.

Die Reagenzien eines erfindungsgemäßen Testkits können in flüssiger oder lyophilisierter Form bereitgestellt werden. Für den Fall, dass einige oder alle Reagenzien des Testkits als Lyophilisate vorliegen, kann das Testkit zusätzlich die zur Lösung der Lyophilisate erforderlichen Lösemittel enthalten, wie z.B. destilliertes Wasser oder geeignete Puffer.

Die folgenden Beispiele dienen der Veranschaulichung der vorliegenden Erfindung und sind nicht als Einschränkung zu verstehen.

### BEISPIELE

### Beispiel 1: Homogener Immunoassay zur Detektion von anti-PF4/Heparin-Komplex-Antikörpern

Die hier verwendete LOCI^{®}-Technologie beruht darauf, dass eine Latexpartikel-gekoppelte Chemilumineszenzverbindung (Chemibeads) und ein Latexpartikel-gekoppelter Photosensibilisator (Sensibeads) durch gleichzeitige Bindung an einen Analyten in eine räumliche Nähe zueinander gebracht werden, so dass Singulett-Sauerstoff, der vom Photosensibilisator erzeugt wird, die Chemilumineszenzverbindung anregen kann.

2 µL einer humanen Plasmaprobe wurden mit 7,5 µL einer Suspension enthaltend humane, gewaschene Thrombozyten von 6 gesunden Spendern (ca. 300 x 10⁹ Thrombozyten/L) und 10 µL einer Pufferlösung enthaltend 0,4 IU/mL Heparin gemischt und bei 37 °C inkubiert. Nach 10 Minuten wurden dem Reaktionsgemisch folgende Komponenten zugegeben:
- 50 µL eines ersten Reagenzes ("Reagenz BA") enthaltend einen ersten biotinylierten monoklonalen Antikörper mit Spezifität für das Neoepitop von F1+2; und anschließend
- 50 µL eines zweiten Reagenzes ("Reagenz Chemibeads") enthaltend einen zweiten monoklonalen Antikörper mit Spezifität für den Immunkomplex bestehend aus dem ersten Antikörper und daran gebundenem F1+2-Peptid, welcher an Latexpartikel gekoppelt ist, die mit einer Chemilumineszenzverbindung assoziiert sind (Chemibeads); und
- 100 µL eines dritten Reagenzes ("Reagenz Sensibeads") enthaltend Streptavidin beschichtete Latexpartikel, die mit einem Photosensibilisator assoziiert sind (Sensibeads).

Nach etwa 10 Minuten wurde das Chemilumineszenzsignal gemessen [kcounts].

Mit dem erfindungsgemäßen Verfahren (im Folgenden auch "F1+2-Generierungstest" genannt) wurden Plasmaproben von 10 HIT-Patienten gemessen, bei denen anhand klinischer Kriterien (4T-Score, zum Teil mit thrombotischem Ereignis) eine HIT diagnostiziert und das Vorliegen von anti-PF4/Heparin-Komplex-Antikörpern mit zwei unabhängigen, kommerziell erhältlichen Immunoassays (HemosIL^{®} AcuStar HIT-Ab(PF4-H), Instrumentation Laboratories und Asserachrom^{®} HPIA-IgG, Diagnostica Stago) festgestellt worden war.

Mit dem erfindungsgemäßen Verfahren wurden ferner Plasmaproben von 7 gesunden Spendern (die keine klinischen HIT-Kriterien und auch keine anti-PF4/Heparin-Komplex-Antikörper aufwiesen) und ein normaler Plasmapool (aus etwa 20 Plasmen gesunder Spender, "FNP") gemessen.

Als hoch-positive Kontrolle für den F1+2-Generierungstest wurde anstelle einer Plasmaprobe eine Antikörperlösung enthaltend einen Thrombozyten-aktivierenden anti-PF4/Heparin-Komplex-Antikörper (50 pg/mL) eingesetzt, um eine hohe Fl+2-Generierung mit dem verwendeten Thrombozyten-haltigen Reagenz zu ermitteln. Eine F1+2-Kalibratorreihe erlaubt eine Quantifizierung der generierten F1+2-Menge in pmol/L.

Die genannten Proben wurden zu Vergleichszwecken außerdem mit dem [14C]-Serotonin-Release Assay (im Folgenden auch "SRA" genannt) gemessen, nach Sheridan, D. et al. (1986) A diagnostic test for heparin-induced thrombocytopenia. Blood 67(1): 27-30, der als Goldstandard gilt.

In Tabelle 1 sind die Testergebnisse zusammengefasst.

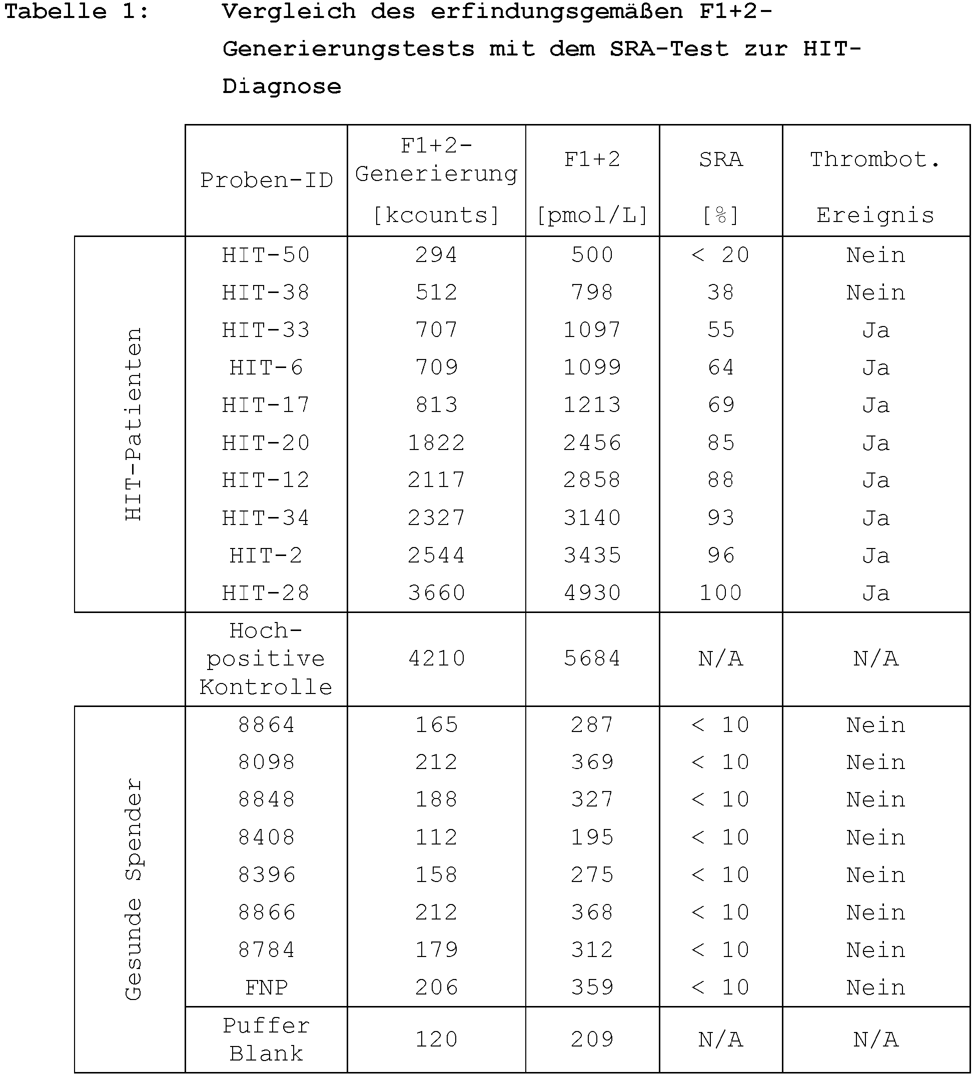

Es zeigt sich, dass die Ergebnisse des erfindungsgemäßen Verfahrens sehr gut mit den Ergebnissen des SRA-Goldstandard-Tests korrelieren. In allen Reaktionsgemischen enthaltend HIT-Patientenproben ist eine gegenüber gesunden Spendern signifikant erhöhte F1+2-Konzentration nachweisbar. Als Grenzwert (Cut-Off) für die Differenzierung von Proben, die anti-PF4/Heparin-Komplex-Antikörper enthalten, von solchen die keine enthalten, könnte ein F1+2-Generierungswert von ≥ 500 pmol/L F1+2 festgelegt werden (Cut-Off SRA-Test: ≥ 20%). Für eine statistisch genauere Festlegung des Cut-Off-Wertes ist jedoch eine deutliche höhere Anzahl von Probenmessungen erforderlich.

Ferner zeigt sich, dass in Reaktionsgemischen enthaltend Proben von HIT-Patienten mit einem thrombotischen Ereignis, im Vergleich zu Reaktionsgemischen enthaltend Proben von HIT-Patienten ohne thrombotisches Ereignis, eine erhöhte Fl+2-Konzentration nachweisbar ist, was einen Hinweis auf einen prokoagulatorischen Status des Patienten darstellt.

## Patentansprüche

1. Verfahren zur Detektion von anti-PF4/Heparin-Komplex-Antikörpern in einer Körperflüssigkeitsprobe, das Verfahren umfassend die Schritte:
i. Bereitstellen eines Reaktionsgemisches durch Vermischen der Probe mit einem Thrombozyten-haltigen Reagenz und mit einem PF4-bindenden, unverzweigten Polysaccharid oder mit einem PF4-bindenden Polyanion oder mit einem Komplex bestehend aus PF4-Protein gebunden an ein unverzweigtes Polysaccharid oder an ein Polyanion;
ii. Inkubation des Reaktionsgemisches; und dann
iii. Bestimmen der Menge von Prothrombinfragment F1+2 (F1+2) in dem Reaktionsgemisch;
iv. Vergleichen der so bestimmten Menge von F1+2 in dem Reaktionsgemisch mit einem vorbestimmten Referenzwert für die Menge von F1+2 in Reaktionsgemischen enthaltend Körperflüssigkeitsproben von Spendern, die bekanntermaßen keine anti-PF4/Heparin-Komplex-Antikörper enthalten; und
v. Feststellen des Vorhandenseins von anti-PF4/Heparin-Komplex-Antikörpern in der Probe, wenn die in dem Reaktionsgemisch bestimmte Menge von F1+2 den Referenzwert überschreitet.

2. Verfahren gemäß Anspruch 1, wobei das Thrombozyten-haltige Reagenz humane Thrombozyten von einem oder mehreren gesunden Spendern, die bekanntermaßen keine anti-PF4/Heparin-Komplex-Antikörper enthalten, enthält.

3. Verfahren gemäß einem der Ansprüche 1 und 2, wobei zum Bestimmen der Menge von F1+2 in dem Reaktionsgemisch mindestens ein anti-F1+2-spezifischer-Antikörper mit dem Reaktionsgemisch in Kontakt gebracht wird.

4. Verfahren gemäß Anspruch 3, wobei
• ein erster und ein zweiter anti-F1+2-Antikörper oder ein erster F1+2-spezifischer Antikörper und ein zweiter Antikörper mit Spezifität für den Immunkomplex bestehend aus F1+2 und dem ersten Antikörper und
• eine erste und eine zweite Komponente eines signalbildenden Systems, welche so zusammenwirken, dass ein detektierbares Signal entsteht, wenn die erste und die zweite Komponente des signalbildenden Systems in räumliche Nähe zueinander gebracht werden,
mit dem Reaktionsgemisch vermischt werden und wobei der erste anti-F1+2-Antikörper mit der ersten Komponente des signalbildenden Systems assoziiert ist oder während der Inkubation des Reaktionsgemisches assoziiert wird und wobei der zweite anti-F1+2- oder Immunkomplexspezifische Antikörper mit der zweiten Komponente des signalbildenden Systems assoziiert ist oder während der Inkubation des Reaktionsgemisches assoziiert wird.

5. Verfahren gemäß Anspruch 4, wobei die erste und zweite Komponente des signalbildenden Systems jeweils eine partikuläre Festphase umfassen, und wobei die Agglutination der partikulären Festphasen im Reaktionsgemisch gemessen wird.

6. Verfahren gemäß Anspruch 4, wobei die erste Komponente des signalbildenden Systems ein chemilumineszierendes Agens ist und die zweite Komponente des signalbildenden Systems ein Photosensitizer ist oder umgekehrt und wobei die Chemilumineszenz im Reaktionsgemisch gemessen wird.

7. Verfahren gemäß einem der Ansprüche 4 bis 6, bei dem das bereitgestellte Reaktionsgemisch keinem
Zentrifugationsschritt zur Abtrennung der Thrombozyten unterworfen wird.

8. Verfahren zur Diagnose oder Prognose einer Heparin-induzierten Thrombozytopenie, wobei mit einem Verfahren gemäß einem der Ansprüche 1 bis 7 das Vorhandensein von anti-PF4/Heparin-Komplex-Antikörpern in einer Körperflüssigkeitsprobe eines Patienten detektiert wird.

9. Testkit zur Durchführung eines Verfahrens gemäß einem der Ansprüche 1 bis 8, welches folgende Komponenten enthält:
a. ein erstes Reagenz enthaltend Thrombozyten;
b. ein zweites Reagenz enthaltend ein PF4-bindendes, unverzweigtes Polysaccharid oder ein PF4-bindendes Polyanion oder einen Komplex bestehend aus PF4-Protein gebunden an ein unverzweigtes Polysaccharid oder an ein Polyanion; und
c. ein oder mehrere Reagenzien zum Nachweis von F1+2, wobei mindestens ein Reagenz einen anti-F1+2-spezifischen Antikörper enthält.

10. Testkit gemäß Anspruch 9, wobei das zweite Reagenz
• ein PF4-bindendes, unverzweigtes Polysaccharid aus der Gruppe Heparin, unfraktioniertes Heparin, fraktioniertes Heparin, Dextransulfat und Fucoidan enthält; oder
• ein PF4-bindendes Polyanion aus der Gruppe Polyvinylsulfat, Polyvinylsulfonat, Polyvinylphosphat, Polyvinylphosphonat, Polystyrensulfat und Polystyrensulfonat; oder
• einen Komplex bestehend aus PF4-Protein gebunden an eines der vorgenannten unverzweigten Polysaccharide oder bestehend aus PF4-Protein gebunden an eines der vorgenannten Polyanionen
enthält.
